# EUROPEAN PATENT APPLICATION

(11) **EP 1 860 116 A1**
(43) Date of publication of application: **28.11.2007**
(21) Application number: 07006461.3
(22) Date of filing: 29.03.2007
(51) Int. Cl.: C07K 9/00, A61K 38/14, A61P 39/06

(54) **Trehalose conjugate with carnosine having antioxidant activity, stable to enzymatic hydrolysis, procedure for its preparation, and pharmaceutical, cosmetic and nutraceutical compositions that contain it**

(30) Priority: 07.04.2006 IT MI20060680
(71) Applicant: Universita' Degli Studi Di Catania, 95124 Catania (IT)
(72) Inventor: Rizzarelli, Enrico, 95124 Catania (IT); Vecchio, Graziella, 95124 Catania (IT); Lazzarino, Giuseppe, 95124 Catania (IT); Amorini, Angela Maria, 95124 Catania (IT); Bellia, Francesco, 95124 Catania (IT)
(74) Representative: Minoja, Fabrizio

(57) **Abstract**

A new conjugate of trehalose with carnosine has been synthesised as a system with antioxidant, antiglycating, chelating and antiaggregant activity. The resistance of the new conjugate to brain dipeptidase and its antioxidant activity have been demonstrated *in vitro.*

## Description

The invention relates to a carnosine derivative (β-alanyl-L-histidine) with formula I, obtained by functionalising α,α'-trehalose with carnosine.

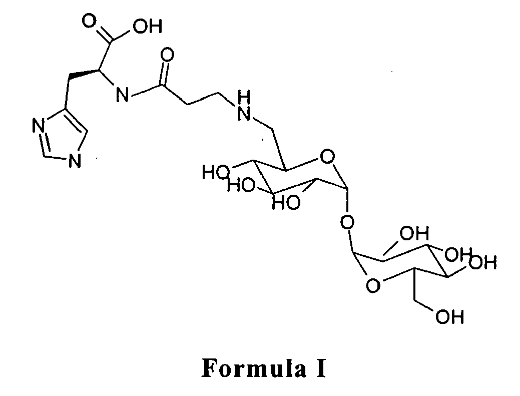

The invention further relates to a process for the preparation of 6-(β-alanyl-L-histidine)-6-deoxy-α,α'-trehalose and formulations containing compound I for pharmaceutical, cosmetic and nutraceutical use with antioxidant, antiglycating, chelating and antiaggregant activity.

### PRIOR ART

The cell respiration process generates an important and potentially dangerous by-product: the superoxide radical, which can react with and damage biological macromolecules like proteins, DNA and lipids, and generate other highly reactive species (ROS) such as ^{·}OH, RO₂^{·}, ROOH RO^{·}, H₂O₂ and ONOO^{·}, all of which are potentially aggressive to cell systems. All aerobic organisms possess defence mechanisms against the radical O₂^{·-}, the main ones being superoxide dismutase (SOD) enzymes which catalyse the dismutation of O₂^{·-} with formation of O₂ and H₂O₂. The hydrogen peroxide produced is transformed in turn into H₂O by catalase and glutathione peroxidase, thus preventing the formation of the radical ^{·}OH, according to the Fenton reaction (Fe(II) + H₂O₂ → Fe(III) + ^{·}OH + OH⁻). Under certain pathological conditions (inflammatory processes, reperfusion ischaemia, heart attack, Alzheimer's disease, ALS, etc.), the SOD enzymes may be insufficient to dismute the excess O₂^{·-} produced, and the biological tissue may suffer further damage in these cases. In view of the higher level of oxidative metabolism characterising some tissue of vertebrates, such as muscle and brain tissue, a high level of small molecules with antioxidant activity has been found, such as glutathione and other dipeptides (carnosine, homocarnosine, anserine, etc.), which have the function of strengthening protection against oxidative stress processes. Protective activity against some ROS has been demonstrated for these peptides (Biochim. Biophys. Acta 1570, 89, 2002; Molecules and Cells 13, 498, 2002; Biochem. J. 967, 241, 1988). For carnosine in particular, as regards its antiradical and antiperoxidative activity, pre-clinical studies demonstrate its ability to prevent and partly cure cataracts (Biochem. Int. 15, 1105; 1987; Biochim. Biophys. Acta 1004, 363, 1989). The anti-inflammatory activity of carnosine has also been demonstrated in a series of tissues: the digestive apparatus, eyes and skin (US4508728, DE4316293, WO 01/52808).

It has also been found that the presence of carnosine, anserine, homocarnosine and other similar peptides improves the intestinal absorption of zinc, increasing its bioavailability. This finding is particularly important in the case of zinc deficiency syndrome and inflammatory intestinal disorders (Biomedical Res. Trace Elements 12, 159, 2001).

The peptide nature of carnosine imposes limitations on its use, associated with breakdown by specific dipeptidases. An attempt has been made to overcome this limitation by using N-acetyl-carnosine (WO 95/10294), which is broken down much more slowly than carnosine (Clinical Chim. Acta 254 (1996) 1-21). Derivatisation of carnosine with cyclodextrins (EP 1176154), compounds widely used as drug carriers, has been employed for this purpose. In this case, the presence of cyclodextrin stabilises the dipeptide, protecting it against hydrolysis by carnosinase, as demonstrated for similar systems (J. Am. Chem. Soc. 120, 7030, 1998), and consequently allows the performance of its biological activity, boosted by the presence of the oligosaccharide, which eliminates the ^{·}OH radicals (Helv. Chim. Acta 85, 1633, 2002).

### DESCRIPTION OF THE INVENTION

The compound according to the invention is obtained by conjugating carnosine with α,α-trehalose, α-D-glucopyranosyl and α-D-glucopyranoside, a disaccharide formed by two glucose molecules. Trehalose, a sugar contained in many living systems, is currently used in the cosmetic field with protective and moisturising functions. Its ability to protect proteins against conformational variations caused by denaturing agents has been demonstrated, with possibly important applications in Huntington's disease (Tibtech 16, 460, 1998; Nat. Med. 10, 148, 2004; J. Mol. Med. 343, 2005).

The compound according to the invention is useful for the treatment of conditions involving protein aggregation processes, such as cataracts or dry eye.

The antioxidant activity of compound I was evaluated *in vitro* by comparison with low-density lipoproteins (LDL) subjected to lipid peroxidation induced by copper (II) ions. The results showed a dose-dependent antioxidant effect, which was significantly greater than that of carnosine. It was also found for the compound according to the invention that carnosinase is more resistant to enzymatic hydrolysis than carnosine.

The presence of trehalose in the conjugate consequently boosts the action against oxidative processes already possessed by carnosine, and guarantees considerable stabilisation of the carnosine residue towards carnosinase, and consequently greater activity than carnosine or simple derivatives thereof, such as N-acetyl carnosine.

According to the invention, the compound with formula (I) was synthesised from suitably modified trehalose and carnosine protected at the carboxyl group. The mono-tosylate in 6 is the preferred derivative of trehalose, while carnosine is preferably used in the form of methyl ester.

Other carnosine esters and/or other activated derivatives of trehalose, equivalent to tosylate, could obviously be used.

The compound with formula I proved to be more resistant to the carnosinase contained in rat brain homogenate than carnosine.

The invention therefore also relates to pharmaceutical, cosmetic or nutraceutical compositions containing compound I as active constituent, mixed with suitable vehicles and excipients.

In view of their antioxidant, antiglycating and transition-metal chelating properties, the compositions according to the invention can be used for the treatment or prevention of conditions in which a pathogenetic role is played by the formation of free radicals or altered protein conformation due to glycation. Examples of these conditions include cataracts, dry eye and skin aging.

The dose and administration route will depend on a number of factors, and will usually be determined by the clinical expert on the basis of the pharmacokinetic and toxicological properties of compound I. Broadly speaking, the doses will be of the same order of magnitude as those described for carnosine (e.g. 100 to 5000 mg a day per os, and concentrations of 0.1 to 10% for topical formulations).

The doses could also be advantageously lower than those of carnosine, due to the greater activity of the compound according to the invention.

Possible formulations include capsules, tablets, granules, powders, solutions, eyewashes and the like.

The following example further illustrates the invention.

Commercial reagents were used unless otherwise indicated, and treated as follows when necessary: trehalose (TH) (Sigma) was dried under vacuum (10⁻² mmHg) for approx. 12 h at 80°C, using a P₂O₅ trap.

Carnosine methyl ester (AHOMe) was synthesised from carnosine (Sigma) with HCl in MeOH at 0°C. Acetyl chloride was used as HCl source.

The tosylate at 6 of trehalose (TosTH) was synthesised from trehalose as follows: trehalose (50 g) was dissolved in pyridine (160 ml) at ambient T. The system was cooled to -35°C, and TosCl (50.2 g) was added under stirring. After 2h the solvent was evaporated and the solid was purified by chromatography on an RP8 column, using a linear gradient of H₂O-MeOH (0-70%) as eluent. Yield 30%, TLC Rf=0.39 BuOH-EtOH-H₂O 5:3:2.

Thin-layer chromatography (TLC) was performed on silica gel (60F-254, 0.25 mm, Merck) and the products, not visible under UV light, were revealed with a 5% solution of anisaldehyde in ethanol (containing 5% H₂SO₄) and Pauli's reagent for peptide derivatives.

The ¹H NMR spectra were recorded on a Varian instrument (Inova 500) at 500 MHz, using the frequency of HDO as reference frequency.

In order to test the antioxidant activity of carnosine and the carbohydrate derivative, freeze-dried human LDL (Sigma) was used as lipid peroxidation target.

The MDA standard was obtained by hydrolysis of the bis-dimethylacetal derivative (Sigma) in 1% sulphuric acid.

### Example

### Synthesis of 6-(β-alanylhistidine)-6-deoxy-α,α'-trehalose

Carnosine methyl ester (in a molar ratio of 1:5 with THTos) was added to a solution of THTos (0.1 g) in anhydrous DMF (1 ml) under N₂ flow. The reaction was carried out a 70°C, under stirring and under nitrogen, and after 14 h the DMF was evaporated under vacuum at 40°C. The solid obtained was purified with a CM-Sephadex C-25 cation-exchange column (in NH₄⁺ form), using water as eluent and a linear gradient of NH₄HCO₃ (0-0.1 M). The fractions collected were analysed by thin-layer chromatography (TLC), and those containing the product were concentrated under vacuum at 40°C. The THAHOMe thus obtained was hydrolysed with 1% NaOH in methanol/water for approx. 1 h. The solid obtained after evaporation of the solvent was further purified with a CM-Sephadex C-25 column using water as eluent. Yield 20%. ¹H NMR (D₂O, 500 MHz) (ppm) 7.72 (s, 1 H, H-2 of the imidazole ring), 6.88 (s, 1 H, H-5 of imidazole), 5.12 (d, 1H, H-1 of TH), 5.07 (d, 1H4, H-1 TH), 3.94 (m, 1 H, C*H* of the histidine chain), 3.94 (m, 1 H, H-5 of TH), 3.88-3.70 (m, 3 H, H-3,-3',-6'_{b}), 3.66 (dd, 1H, H-6'a), 3.58 (dd, 1H, H-2), 3.52 (dd, 1H, H-2'), 3.34 (t, 1H, H-4), 3.30 (dd, 1H, H-6_{b}), 3.25 (m, 2H, H-6ₐ, H-'4), 3.10 (m, 2 H, CH₂ of the ethylene chain in α to the NH group); 3.04 (m, 1 H, H of CH₂ of His), 2.87 (dd, 1 H, another proton of CH₂ of His), 2.26 (m, 2H, CH₂ of the ethylene chain in α to the carbonyl group).

### Antioxidant activity towards low-density lipoproteins (LDL)

The process of peroxidation of humane LDLs (Sigma), suspended in 20 mM ammonium acetate at pH 7.4, was triggered by the addition of Cu²⁺ (40 µM) to the protein suspension, followed by a 4 h incubation at 37°C (Free Radic. Res. 35, 953, 2001; J. Agr. Food Chem. 45, 3362, 1997). The lipid peroxidation inhibiting effect of the trehalose-carnosine conjugate was evaluated by adding this molecule to the LDL-Cu²⁺ system at increasing concentrations (5, 10, 20, 50, 100 and 200 µM). The peroxidation reaction was interrupted by adding an equal volume of acetonitrile, followed by double extraction with chloroform.

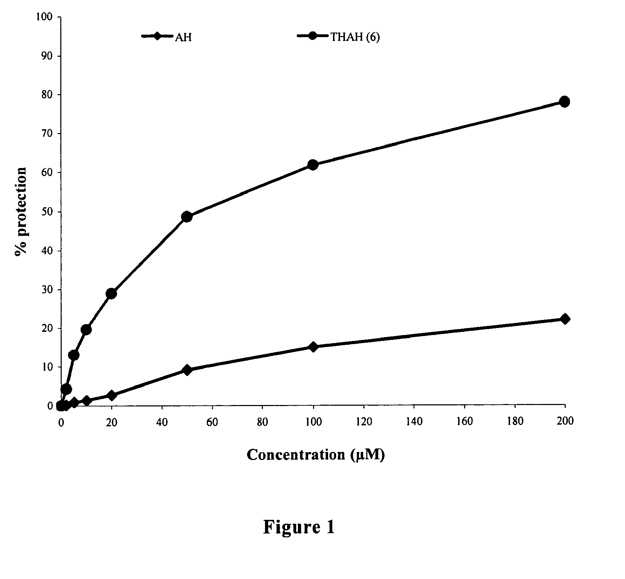

For the determination of MDA, the end product of lipid peroxidation, the aqueous extract was analysed by HPLC, using an analysis method reported in Anal. Biochem. 197, 191, 1991 (data reported in figure 1).

### Enzymatic hydrolysis by brain carnosinase

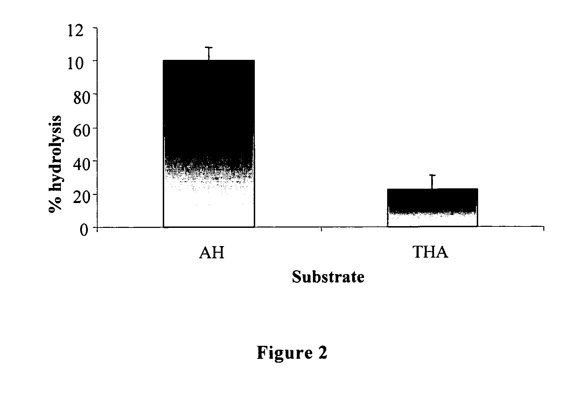

The breakdown of the carbohydrate conjugate of carnosine was measured by the following method: a 20% homogenate of rat brain was prepared with a 50 mM Tris buffer (Tris-(hydroxymethyl)-aminomethane) pH 8.1 containing 2 mM DTT (di-thio-threitol), and the mixture was centrifuged for 20 min at 19000 rpm. Part of the supernatant solution (192 µl) was incubated at 37°C for 2 h with the carnosine-trehalose conjugate, in a final volume of 300 µl. The reaction was interrupted by adding acetonitrile (300 µL), and the solution was subjected to double extraction with chloroform. The amount of histidine in the final solution was quantified fluorometrically after reacting with orthophthaldialdehyde OPA (Fluka), according to a method reported in Clin. Chim. Acta, 1982, 123, 221 (data reported in figure 2).

## Claims

1. Compound with formula I 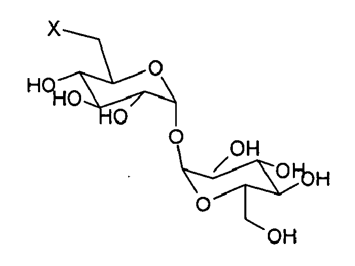 wherein X is the dipeptide carnosine.

2. Process for the preparation of the compound with formula I, **characterised in that** a trehalose derivative is reacted with carnosine protected on the carboxyl.

3. Process as claimed in claim 2, **characterised in that** the monotosylate derivative in position 6 is used as trehalose derivative.

4. Pharmaceutical, cosmetic or nutraceutical compositions containing the compound with formula I as active ingredient.

5. Use of the compound claimed in claim 1 to prepare a composition with antioxidant (radical scavenging), anticataract, transition-metal chelating, antiglycating and antiaggregant activity against protein conformation disorders.
